# EUROPEAN PATENT APPLICATION

(11) **EP 3 919 042 A1**
(43) Date of publication of application: **08.12.2021**
(21) Application number: 20204283.4
(22) Date of filing: 28.10.2020
(51) Int. Cl.: A61K 8/04, A01N 31/02, A61K 8/22, A61K 8/25, A61K 8/34, A61K 8/58, A61P 31/04, A61Q 17/00, A61Q 19/00

(54) **COMPOSITION WITH SILANOL, ETHANOL AND WATER FOR GLOBAL DISINFECTION USE**

(30) Priority: 04.06.2020 EP 20178310
(71) Applicant: Sil'Innov scrl, 6180 Courcelles (BE)
(72) Inventor: Coste-Manière, Ivan, 06130 Grasse (FR); Marzouk, Rada, 13001 Marseille (FR)
(74) Representative: Brantsandpatents bvba

(57) **Abstract**

The present invention provides a composition comprising silanol in an amount of between 0.01 and 10% by weight per volume, ethanol, water and optionally glycerine for use in the global disinfection of human skin and/or human nails, and/or of surfaces.

## Description

### TECHNICAL FIELD

The present invention relates to the field of compositions for global disinfection use and more specifically to such compositions comprising silanol, ethanol and water.

### INTRODUCTION

The presence of water is a crucial factor in destroying or inhibiting microorganisms with the disinfecting agent ethanol. Water acts as a catalyst and plays a key role in denaturing vegetative cell membrane proteins. The inclusion of water in ethanol disinfectants however results in physical effects negatively affecting human skin.

Disinfecting compositions, e.g. disinfecting gels, are described in the state of the art. WO2012088758A1 discloses a gel composition for air freshening or disinfecting, comprising a silicon alkoxide or colloidal silica, a volatile or gaseous fragrance and/or disinfectant, water, an acid or base catalyst, a water-soluble solvent, and optionally other volatile components and additives, and a method for preparing the same. WO2012088758A1, however, does not address the above-mentioned problem regarding the physical effects negatively affecting human skin.

The present invention aims to resolve at least the problem mentioned above.

### SUMMARY OF THE INVENTION

In a first aspect, the present invention provides a composition for use in the global disinfection of human skin and/or human nails, and/or of surfaces, according to claim 1. Preferred embodiments of the composition are presented in claims 2 to 11.

In a second aspect, the present invention provides a kit comprising one or more spatially separated components to be used in a composition for use in the global disinfection of human skin and/or human nails, and/or of surfaces, according to claim 12. Preferred embodiments of the composition are presented in claims 13 and 14.

In a third aspect, the present invention provides a use of a composition according to the first aspect of the invention for multiple daily global hand-disinfection against harmful microorganisms, according to claim 15.

In a fourth aspect, the present invention provides a use of a composition according to the first aspect of the invention for multiple daily global disinfection of surfaces, like tables and kitchen counters.

In a fifth aspect, the present invention provides a use of a composition according to the first aspect of the invention in long-release compositions or in galenic forms.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
The recitation of numerical ranges by endpoints includes all numbers and fractions subsumed within that range, as well as the recited endpoints.

The term "antimicrobial agents" as used herein refers to any naturally-occurring, synthetic, or semi-synthetic compound or composition or mixture thereof, which is safe for human use, and is effective in killing or substantially inhibiting the growth of microbes. "Antimicrobial" as used herein, includes antibacterial, antifungal, and antiviral agents.

The term silanol is to be understood as the general name for a family of chemical compounds containing the element silicon attached to at least one hydroxyl group. A silanol is the group of chemicals characterized by the functional group Si-OH.

In a preferred embodiment, the silanol is chosen from the list of silicic acids and organosilanols.

The term "silicic acid" is to be understood as is the general name for a family of chemical compounds containing the element silicon attached to oxide and hydroxyl groups. This family of compounds has the general formula [SiOₓ(OH)_{4-2*x*}]*ₙ*. Examples include *metasilicic acid* (H₂SiO₃), i.e. the chain or cyclic [SiO(OH)₂]ₙ, *orthosilicic acid* (H₄SiO₄, i.e. Si(OH)₄ with calculated p*K*_{*a*1}= 9.84, p*K*_{*a*2}=13.2 at 25 °C), *disilicic acid* (H₂Si₂O₅), i.e. the polymer [SiO_{1.5}(OH)]ₙ, and *pyrosilicic acid* (HeSi₂O₇), i.e. O(Si(OH)₃)₂. Preferably, said silicic acid is orthosilicic acid. The inventors have found that in the context of the present invention orthosilicic acid provides the best performance in the intended uses.

The term "organosilanol" is to be understood as the general name for a family of chemical compounds containing the element silicon attached to at least one hydroxyl group and further comprising silicon-carbon bonds. This family of compounds has the general formula SiRₓ(OH)₄₋ₓ, wherein Rₓ are independently selected from are independently selected from H, alkyl, alkenyl, alkynyl, phenyl and alkoxy groups. In a preferred embodiment, Rₓ are independently selected from H, alkyl and alkoxy groups. More preferably, Rₓ are independently selected from H, C1-C8 alkyl and C1-C8 alkoxy. More preferably, Rₓ are independently selected from H and C1-C4 alkyl. Examples include Et₃SiOH, MeEt₂SiOH, Me₃SiOH (TMS) and MeSiOH₃ (MMST).

The expression "% by weight per volume", here and throughout the text unless otherwise defined, refers to the relative weight of the respective component based on the overall volume of the composition.

As used herein, the term "pharmaceutically-acceptable carrier" means a chemical composition with which an appropriate compound or derivative can be combined and which, following the combination, can be used to administer the appropriate compound to a subject.

The term "skin" as used herein, refers to the commonly used definition of skin, e.g., the epidermis and dermis, and the cells, glands, mucosa, and connective tissue which comprise the skin.

Other components such as preservatives, antioxidants, surfactants, absorption enhancers, viscosity enhancers or film forming polymers, bulking agents, diluents, colouring agents, flavouring agents, pH modifiers, sweeteners or taste-masking agents may also be incorporated into a composition according to the invention. Suitable colouring agents include, but are not limited to, red, black, and yellow iron oxides and FD&C dyes such as FD&C Blue No. 2 and FD&C Red No. 40. Suitable flavouring agents include mint, raspberry, licorice, orange, lemon, grapefruit, caramel, vanilla, cherry grape flavours and combinations thereof. Suitable pH modifiers include, but are not limited to, citric acid, tartaric acid, phosphoric acid, hydrochloric acid, maleic acid and sodium hydroxide. Suitable sweeteners include, but are not limited to, aspartame, acesulfame K and thaumatin. Suitable taste-masking agents include, but are not limited to, sodium bicarbonate, vanilla, ionexchange resins, cyclodextrin inclusion compounds and adsorbates.

The first aspect of the present invention relates to a composition comprising a silanol in an amount of between 0.01 and 10% by weight per volume, preferably of between 0.013 and 8% by weight per volume, more preferably of between 0.017 and 5% by weight per volume, even more preferably of between 0.02 and 2% by weight per volume and yet even more preferably of between 0.023 and 0.5% by weight per volume, ethanol, water and optionally glycerine for use in the global disinfection of human skin and/or human nails, preferably the skin and/or nails of human hands, and/or of surfaces.

When a human disinfects human skin and/or human nails, preferably the skin and/or nails of human hands, and/or of surfaces, like tables and kitchen counters, a composition comprising both ethanol and water is most effective for global disinfection purposes. In the composition, water acts as a catalyst and plays a key role in denaturing vegetative cell membrane proteins of microorganisms. The inclusion of water in ethanol disinfectants however results in physical effects negatively affecting human skin and/or nails. In comparison with such disinfectant composition comprising only ethanol, the inclusion of water leads to a negative hydroscopic effect on the human skin and/or nails which damages the skin and/or nails. The included silanol, according to said amounts, is selected in order to protect human skin and/or nails against said negative hydroscopic effect resulting from the combination of water and alcohol in the composition. Accordingly, damage of human hands and/or nails by application of the composition can be cured or even prevented by including said silanol. Even when applied to a surface, like tables and kitchen counters, the skin and/or nails protecting effect of silanol is of relevance, since the composition will normally be applied to a surface by means of a human hand, albeit a human hand operating a receptacle including the composition.

Said composition may further comprise one or more pharmaceutically acceptable excipients and/or emollients. Non-limiting examples of suitable excipients are animal and vegetable fats, oils, waxes, paraffins, starch, tragacanth, cellulose derivatives, polyethylene glycols, silicones, bentonites, talc and zinc oxide, or mixtures thereof.

In a preferred embodiment of a composition according to the first aspect of the invention, ethanol is comprised in the composition in an amount of between 65 and 85% by weight per volume, more preferably of between 67 and 83% by weight per volume and more preferably of between 69 and 81% by weight per volume of the composition and water is comprised in the composition in an amount of between 5 and 25% by weight per volume, more preferably of between 10 and 20% by weight per volume, more preferably of between 12 and 18% by weight per volume and yet even more preferably of between 14 and 16% by weight per volume. Said amounts of ethanol and water in the composition are optimally suited for global disinfection purposes, and at the same time optimally allow, in combination with above-mentioned amounts of silanol, good health of human skin and/or nails.
In a preferred embodiment of a composition according to the first aspect of the invention, the composition comprises glycerine in an amount of between 0.5 and 3.5% by weight per volume, more preferably of between 1.0 and 3.0% by weight per volume, more preferably of between 1.5 and 2.5% by weight per volume and even more preferably of between 1.75 and 2.25% by weight per volume. Glycerine acts as a lubricating agent in the composition, thereby providing the solution with reduced friction and wear properties when applied to human skin and/or human nails, preferably the skin and/or nails of human hands, and/or of surfaces. Glycerine, and most effectively when used in the above-mentioned amounts, is a most suitable lubricant because of its property of high viscosity and ability to remain fluid at low temperatures.

In a preferred embodiment of a composition according to the first aspect of the invention, the composition comprises hydrogen peroxide in an amount of between 0.05 and 0.5% by weight per volume, more preferably of between 0.1 and 0.45% by weight per volume and even more preferably of between 0.15 and 0.4% by weight per volume. Hydrogen peroxide is an effective antimicrobial agent and can be used in the composition as an additional compound with disinfecting properties. For this purpose, the above-mentioned amounts of hydrogen peroxide are optimally suited.

In a preferred embodiment of a composition according to the first aspect of the invention, the composition comprises at least one stabilizing agent based on phenol or polyphenol, and silicic acid has free hydroxyl groups, the at least one stabilizing agent stabilizing said free hydroxyl groups of silicic acid, wherein a weight ratio between a Si content of the composition and the content of stabilizing agent is of between 0.1 and 1. By said stabilization, a stable silicon complex is formed. Without being bound to any mechanistic studies, it is assumed that said silicic acid is stabilized by hydrogen bonding and optionally further interactions.

In a preferred embodiment of a composition according to the first aspect of the invention, said silicic acid is orthosilicic acid having four free hydroxyl groups. Orthosilicic acid provides improved results in terms of use as human skin and/or nails protecting agent. In a silicon complex of orthosilicic acid and the at least one stabilizing agent based on phenol or polyphenol, the orthosilicic acid carries four free hydroxyl groups, which excludes any presence of organic radical and therefore of organosilanes.

The orthosilicic acid is preferably complexed with at least one phenolic or polyphenolic compound comprising at least one aromatic ring and one or more hydroxyl groups. Preferably it could further contain one or more carbonyl groups (C=O). The aromatic, and therefore sterically hindered, structure of phenolic or polyphenolic compounds and the presence of hydroxyl and optionally carbonyl groups play a fundamental role in the stabilization process. Hydrogen bonds characteristic of weak electrostatic bonds are established between the hydroxyl groups of orthosilicic acid and the hydroxyl and carbonyl groups of the phenolic compounds. Although not being bound by any mechanistic theories, it is assumed that an esterification reaction between the orthosilicic acid and the phenolic compound (polyphenolic) is excluded. These bonds prevent the polymerization of orthosilicic acid and the formation of Si-O-Si bonds.

In a preferred embodiment of a composition according to the first aspect of the invention, said composition is comprised in a topical or transdermal dosage form. Dosage forms for topical or transdermal administration of a composition according to the first aspect of the invention include liquids, ointments, pastes, creams, lotions, gels, solutions, sprays, aerosols or patches. Silanol is preferably admixed under sterile conditions with a pharmaceutically acceptable carrier and any needed preservatives or buffers as may be required. Such dosage forms can be made by dissolving or dispensing the compound(s) in the proper medium. Absorption enhancers can also be used to increase the flux of silanol across the skin and/or nails. The rate can be controlled by either providing a rate controlling membrane or by dispersing the compound in a polymer matrix or gel.

In a preferred embodiment of a composition according to the first aspect of the invention, said stabilizing agent is selected from the group comprising phenolic acids, phenols, aldehyde derivatives of phenolic acids, cinnamic aldehydes, coumarins, naphthoquinones, flavonoids, stilbenes and mixtures of one or more of the aforementioned. The stabilizer is preferably a phenolic or polyphenolic compound and the following compounds, not limited thereto, may be exemplified: phenolic acids, such as 3-4-5-trihydroxybenzoic acid, 4-hydroxy-3- acid; methoxybenzoic acid, 3-4-dihydroxybenzoic acid, 4-hydroxy-3,5-dimethoxybenzoic acid, 2-hydroxybenzoic acid, 2-5-dihydroxybenzoic acid, 3-(4-hydroxyphenyl) acid 2-propenoic acid, 3-(3,4-dihydroxyphenyl) prop-2-enoic acid, 3-(4-hydroxy-3-methoxyphenyl) prop-2-enoic acid, 3-(4-hydroxy-3,5-dimethoxyphenyl)-prop-2-enoic acid, (R)-a-[[3-(3,4-dihydroxyphenyl)-1-oxo-2E-propenyl]-oxy]-3,4-dihydroxybenzenepropanoic acid, etc.; simple phenols, such as benzene-1,2-diol, benzene-1,3-diol, 2-isopropyl-5-methylphenol; phenylbutanone, such as 1-(4-hydroxyphenyl) -3-butanone; aldehyde derivatives of phenolic acids, such as 4-hydroxy-3-methoxybenzaldehyde, etc.; cinnamic aldehydes, such as 4-allyl-2-methoxyphenol, 2-methoxy-4-propenylphenol, etc.; coumarins, such as 7-hydroxychromen-2-one, 6,7-dihydroxychromen-2-one, etc.; naphthoquinones, such as 5-hydroxy-1,4-naphthoquinone, etc.; flavonoids, such as catechin (2-(3,4-dihydroxyphenyl) chroman-3,5,7-triol) and epicatechin (EC), epigallocatechin (EGC), epicatechin gallate (ECG), epigallocatechin gallate (EGCG), kaempferol, quercetol, luteolin, etc.; stilbenes, such as resveratrol, pinosylvin, piceatannol, pterostilbene, etc. Preferred stabilizers are 4-hydroxy-3-methoxybenzaldehyde, 1-(4-hydroxyphenyl)-3-butanone, 2-hydroxybenzoic acid, and (2-(3,4-dihydroxyphenyl)-chromane-3,5,7-triol). In a more preferred embodiment, the first aspect of the invention provides a composition according to the first aspect of the invention, wherein said stabilizing agent is 4-hydroxy-3-methoxybenzaldehyde.

In a preferred embodiment of a composition according to the first aspect of the invention, the composition comprises monomethylsilanetriol (MMST). Monomethylsilanetriol (MMST, MeSiOH₃ or Si(OH)₃CH₃) is an organosilanol. The inventors have found that monomethylsilanetriol provides improved results. This organosilanol is highly bioavailable. Furthermore, MMST is sufficiently stable in water at low concentrations, unlike orthosilicic acid which tends to form silica gel due to polymerization of orthosilicic acid in water, thereby decreasing its bioavailability. MMST can advantageously be used without stabilizers in silicon equivalent concentrations up to 300 mg/l. The stability of MMST is dependent on concentration and declines at higher concentrations. The stability can further be enhanced by the use of stabilizing agents. In a preferred embodiment the stabilizing agent is based on phenol or polyphenol as described above. The same reasoning applies, mutatis mutandis, with respect to stabilizing effect on the free hydroxyl groups.

In a preferred embodiment of a composition according to the first aspect of the invention, the composition comprises an organosilanol preferably monomethylsilanetriol in combination with an antioxidant. Antioxidants are beneficial to neutralize the presence of organic radicals. Organic radicals may be formed due to organosilanol decomposition.
In a preferred embodiment of a composition according to the first aspect of the invention, the composition further comprises one or more therapeutic agents, preferably selected from the group consisting of vitamins, antimicrobial agents, antifungal agents, anti-inflammatories, wound care products, wound healing agents, plant extracts, antibacterial agents, antifungal agents, antiviral agents, and/or antibiotics.

The second aspect of the present invention relates to a kit comprising one or more spatially separated components to be used in a composition for use in the global disinfection of human skin and/or human nails, and/or of surfaces, the kit comprising a silanol, ethanol, water and optionally glycerine. Preferably, the kit comprises glycerine.

In a preferred embodiment of a kit according to the second aspect of the invention, the kit comprises hydrogen peroxide.

In a preferred embodiment of a kit according to the second aspect of the invention, the kit comprises one or more spatially separated components according to a composition according to the first aspect of the invention.

For the technical effects and advantages and/or preferred embodiments of the features of the kit according to the second aspect of the invention, reference is made to the embodiments of the first aspect of the invention referred above in which corresponding features are described and which are also applicable to the kit according to the second aspect of the invention.

In a particular embodiment, the kit comprises the silanol compound at a pH lower than 7, preferably lower a pH lower than 6, more preferably lower a pH lower than 5, more preferably lower a pH lower than 4, more preferably lower a pH lower than 3. In the a preferred embodiment, the kit comprises the silanol compound at a pH between 1 and 4, more preferably between 2 and 4. One advantage specific to the kit is long term storage. Silicon compounds including silanol compounds may polymerize forming Si-O-Si stable bonds, eventually forming a wet silica gel. This reaction is generally favoured in an alkali solution and hindered in an acidic solution. A kit comprising the silanol compound in acidic solution is stable almost indefinitely. By diluting or neutralizing the acidic, silanol comprising part of said kit, a consumer does not come into contact with an acidic composition yet the kit maintains its prolonged shelflife.

The preferred embodiments of the present invention overcome many of these issues through the choice of surprisingly stable silanol compounds in suitable concentrations. This is further aided by the addition of stabilizing agents as described previously.

The third aspect of the present invention relates to a use of a composition according to the first aspect of the invention for multiple daily global hand-disinfection against harmful microorganisms. Harmful microorganisms refer to microorganisms harmful for human health.

The fourth aspect of the present invention relates to a use of a composition according to the first aspect of the invention for multiple daily global disinfection of surfaces, like tables and kitchen counters.

The fifth aspect of the present invention relates to a use of a composition according to the first aspect of the invention in long-release compositions or in galenic forms.

In a preferred embodiment of a use according to the fifth aspect of the invention, the composition according to the first aspect of the invention is microencapsulated. Microencapsulated is a preferred technique to allow for long release of the composition according to the first aspect of the invention.

In a preferred embodiment of a use according to the fifth aspect of the invention, the long-release compositions or galenic forms are used for global disinfection of surfaces, like surfaces related to numerous existing sport practices and multiple tasks in everyday life, such as surfaces of sport balls, driving suits or horse saddles. Other examples of said surfaces are tables and kitchen counters.

### EXAMPLES

The present invention will now be further exemplified with reference to the following examples. The present invention is in no way limited to the given examples. For advantages and technical effects of the various elements described below in the examples, reference is made to the advantages and technical effects of corresponding elements described above in the detailed description.

### EXAMPLE 1

Example 1 relates to a composition according to preferred embodiments of the first aspect of the invention that is comprised in a gel. The gel serves as a disinfecting gel for external use for global disinfection of human skin and/or human nails, preferably the skin and/or nails of human hands, and/or of surfaces. The product properties and product composition of a gel according to Example 1 can be found below in Tables 1 and 2, respectively.

**Table 1 Product properties of a gel according to Example 1 comprising a composition according to preferred embodiments of the first aspect of the invention**

| | *Unit* | *Specification* |
|---|---|---|
| *Physical properties* | | |
| Visual aspect | | Clear, colourless or coloured liquid |
| Odour | | Typical |
| Taste | | - |
| Density (at 20 °C) | kg/l | 0.80-0.90 |
| *Chemical properties* | | |
| Ethanol content | %weight per volume | 67-83 |
| Si content | %weight per volume | 0.01-2.4 |

| *Microbiological data* | | |
|---|---|---|
| Salmonella spp. | | absent |
| Enterobacteriaceae | CFU/g | ≤ 10 |
| Other bacteria | CFU/g | ≤ 10 |
| Mould and yeasts | CFU/g | ≤ 10 |

**Table 2 Product composition of a gel according to Example 1 comprising a composition according to preferred embodiments of the first aspect of the invention**

| *Component* | *Amount (*% *weight per volume)* |
|---|---|
| ethanol | 67 - 83 |
| glycerine | 1 - 3 |
| silanol | 0.01 - 8 |
| water | 10 - 20 |

The gel according to Example 1 may be packaged in suitable receptacles, like plastic, preferably polyethylene, receptacles with a desired volume.

The gel according to Example 1 has a minimum shelf life of 24 months after production and is best stored in a cool, dry place in the original receptacle.

### EXAMPLE 2

Example 2 relates to a composition according to preferred embodiments of the first aspect of the invention that is comprised in a gel. The gel serves the same purpose as disclosed for the gel of Example 1. The product properties and product composition of a gel according to Example 2 can be found below in Tables 3 and 4, respectively.

**Table 3 Product properties of a gel according to Example 2 comprising a composition according to preferred embodiments of the first aspect of the invention**

| | *Unit* | *Specification* |
|---|---|---|
| *Physical properties* | | |
| Visual aspect | | Clear, colourless or coloured liquid |
| Odour | | Typical |
| Taste | | - |
| Density (at 20 °C) | kg/I | 0.80 - 0.90 |

| *Chemical properties* | | |
|---|---|---|
| Ethanol content | %weight per volume | 67 - 83 |

| Si content | %weight per volume | 0.01 - 2.4 |
|---|---|---|
| *Microbiological data* | | |
| Salmonella spp. | | absent |
| Enterobacteriaceae | CFU/g | ≤ 10 |
| Other bacteria | CFU/g | ≤ 10 |
| Mould and yeasts | CFU/g | ≤ 10 |

**Table 4 Product composition of a gel according to Example 2 comprising a composition according to preferred embodiments of the first aspect of the invention**

| *Component* | *Amount (% weight per volume)* |
|---|---|
| ethanol | 67 - 83 |
| glycerine | 1 - 3 |
| orthosilicic acid | 0.01 - 8 |
| 4-hydroxy-3-methoxybenzaldehyde | 0.01 - 4 |
| water | 10 - 20 |

The gel according to Example 2 may be packaged in suitable receptacles, like plastic, preferably polyethylene, receptacles with a desired volume.

The gel according to Example 2 has a minimum shelf life of 24 months after production and is best stored in a cool, dry place in the original receptacle.

### EXAMPLE 3

Example 3 relates to a composition according to preferred embodiments of the first aspect of the invention that is comprised in a spray. The spray serves as a disinfecting spray with antiseptic properties for external use for global disinfection of human skin and/or human nails, preferably the skin and/or nails of human hands, and/or of surfaces. The product properties and product composition of a spray according to Example 3 can be found below in Tables 5 and 6, respectively.

**Table 5 Product properties of a spray according to Example 3 comprising a composition according to preferred embodiments of the first aspect of the invention**

| | *Unit* | *Specification* |
|---|---|---|
| *Physical properties* | | |
| Visual aspect | | Clear, colourless or coloured liquid |
| Odour | | Typical |
| Taste | | - |
| Density (at 20 °C) | kg/l | 0.80 - 0.90 |

| *Chemical properties* | | |
|---|---|---|
| Ethanol content | %weight per volume | 67 - 83 |
| Si content | %weight per volume | 0.01 - 2.4 |
| Hydrogen peroxide | %weight per volume | 0.1 - 0.45 |

| *Microbiological data* | | |
|---|---|---|
| Salmonella spp. | | absent |
| Enterobacteriaceae | CFU/g | ≤ 10 |
| Other bacteria | CFU/g | ≤ 10 |
| Mould and yeasts | CFU/g | ≤ 10 |

**Table 6 Product composition of a spray according to Example 3 comprising a composition according to preferred embodiments of the first aspect of the invention**

| *Component* | *Amount (% weight per volume)* |
|---|---|
| ethanol | 67 - 83 |
| glycerine | 1 - 3 |
| silanol | 0.01 - 8 |
| hydrogen peroxide | 0.1 - 0.45 |
| water | 10 - 20 |

The spray according to Example 3 may be packaged in suitable receptacles, like plastic, preferably polyethylene, receptacles with a desired volume.

The spray according to Example 3 has a minimum shelf life of 24 months after production and is best stored in a cool, dry place in the original receptacle.

### EXAMPLE 4

Example 4 relates to a composition according to preferred embodiments of the first aspect of the invention that is comprised in a spray. The spray serves the same purpose as disclosed for the spray of Example 3. The product properties and product composition of a spray according to Example 4 can be found below in Tables 7 and 8, respectively.

**Table 7 Product properties of a spray according to Example 4 comprising a composition according to preferred embodiments of the first aspect of the invention**

| | *Unit* | *Specification* |
|---|---|---|
| *Physical properties* | | |
| Visual aspect | | Clear, colourless or coloured liquid |
| Odour | | Typical |
| Taste | | - |
| Density (at 20 °C) | kg/l | 0.80 - 0.90 |
| *Chemical properties* | | |
| Ethanol content | %weight per volume | 67 - 83 |
| Si content | %weight per volume | 0.01 - 2.4 |
| Hydrogen peroxide | %weight per volume | 0.1 - 0.45 |

| *Microbiological data* | | |
|---|---|---|
| Salmonella spp. | | absent |
| Enterobacteriaceae | CFU/g | ≤ 10 |
| Other bacteria | CFU/g | ≤ 10 |
| Mould and yeasts | CFU/g | ≤ 10 |

**Table 8 Product composition of a spray according to Example 4 comprising a composition according to preferred embodiments of the first aspect of the invention**

| *Component* | *Amount (% weight per volume)* |
|---|---|
| ethanol | 67 - 83 |
| glycerine | 1 - 3 |
| orthosilicic acid | 0.01 - 8 |
| 4-hydroxy-3-methoxybenzaldehyde | 0.01 - 4 |
| hydrogen peroxide | 0.1 - 0.45 |
| water | 10 - 20 |

The spray according to Example 4 may be packaged in suitable receptacles, like plastic, preferably polyethylene, receptacles with a desired volume.

The spray according to Example 4 has a minimum shelf life of 24 months after production and is best stored in a cool, dry place in the original receptacle.

### EXAMPLE 5

Example 5 relates to a composition according to preferred embodiments of the first aspect of the invention that is comprised in a gel. The gel serves the same purpose as disclosed for the gel of Example 1 and 2. The product properties and product composition of a gel according to Example 5 can be found below in Tables 9 and 10, respectively.

**Table 9 Product properties of a gel according to Example 5 comprising a composition according to preferred embodiments of the first aspect of the invention**

| | *Unit* | *Specification* |
|---|---|---|
| *Physical properties* | | |
| Visual aspect | | Clear, colourless or coloured liquid |
| Odour | | Typical |
| Taste | | - |
| Density (at 20 °C) | kg/l | 0.80-0.90 |
| *Chemical properties* | | |
| Ethanol content | %weight per volume | 67-83 |
| Si content | %weight per volume | 0.01-2.8 |
| *Microbiological data* | | |
| Salmonella spp. | | absent |
| Enterobacteriaceae | CFU/g | ≤ 10 |
| Other bacteria | CFU/g | ≤ 10 |
| Mould and yeasts | CFU/g | ≤ 10 |

**Table 10 Product composition of a gel according to Example 5 comprising a composition according to preferred embodiments of the first aspect of the invention**

| *Component* | *Amount (% weight per volume)* |
|---|---|
| ethanol | 67 - 83 |
| glycerine | 1 - 3 |
| monomethylsilanetriol | 0.01 - 8 |
| water | 10 - 20 |

The gel according to Example 5 may be packaged in suitable receptacles, like plastic, preferably polyethylene, receptacles with a desired volume.

The gel according to Example 5 has a minimum shelf life of 24 months after production and is best stored in a cool, dry place in the original receptacle.

## Claims

1. A composition comprising silanol in an amount of between 0.01 and 10% by weight per volume, ethanol, water and optionally glycerine for use in the global disinfection of human skin and/or human nails, and/or of surfaces.

2. Composition according to claim 1, wherein ethanol is comprised in the composition in an amount of between 65 and 85% by weight per volume of the composition and wherein water is comprised in the composition in an amount of between 5 and 25% by weight per volume.

3. Composition according to claim 1 or 2, wherein the composition comprises glycerine in an amount of between 0.5 and 3.5% by weight per volume.

4. Composition according to any of claims 1 to 3, wherein the composition comprises hydrogen peroxide in an amount of between 0.05 and 0.5% by weight per volume.

5. Composition according to any of claims 1 to 4, wherein the composition comprises at least one stabilizing agent based on phenol or polyphenol, and said silanol is a silicic acid having free hydroxyl groups, the at least one stabilizing agent stabilizing said free hydroxyl groups of silicic acid, wherein a weight ratio between a Si content of the composition and the content of stabilizing agent is of between 0.1 and 1.

6. Composition according to claim 5, wherein said silicic acid is orthosilicic acid having four free hydroxyl groups.

7. Composition according to any of claims 5 to 6, wherein said stabilizing agent is selected from the group comprising phenolic acids, phenols, aldehyde derivatives of phenolic acids, cinnamic aldehydes, coumarins, naphthoquinones, flavonoids, stilbenes and mixtures of one or more of the aforementioned.

8. Composition according to claim 7, wherein said stabilizing agent is 4-hydroxy-3-methoxybenzaldehyde.

9. Composition according to any of claims 1-4, wherein said silanol is an organosilanol, preferably monomethylsilanetriol.

10. Composition according to any of claims 1 to 9, wherein said composition is comprised in a topical or transdermal dosage form.

11. Composition according to any of claims 1 to 10, further comprising one or more therapeutic agents, preferably selected from the group consisting of vitamins, antimicrobial agents, antifungal agents, anti-inflammatories, wound care products, wound healing agents, plant extracts, antibacterial agents, antifungal agents, antiviral agents, and/or antibiotics.

12. Kit comprising one or more spatially separated components to be used in a composition for use in the global disinfection of human skin and/or human nails, and/or of surfaces, the kit comprising silanol, ethanol, water and optionally glycerine.

13. Kit according to claim 12, wherein the kit comprises hydrogen peroxide.

14. Kit according to claim 12 or 13, comprising one or more spatially separated components according to a composition according to any of claims 1 to 11.

15. Use of a composition according to any of claims 1 to 11 for multiple daily global hand-disinfection against harmful microorganisms.
